Europäisches Patentamt

European Patent Office

Office européen des brevets

(19)

(11) **EP 0 997 138 A1**

(12) **DEMANDE DE BREVET EUROPEEN**

(43) Date de publication:
**03.05.2000 Bulletin 2000/18**

(51) Int Cl.[7]: **A61K 7/48**, A61K 7/02

(21) Numéro de dépôt: **99401777.0**

(22) Date de dépôt: **15.07.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **11.09.1998   FR 9811360**

(71) Demandeur: **L'OREAL**
**75008 Paris (FR)**

(72) Inventeur: **Ferrari, Véronique**
**94700 Maisons-Alfort (FR)**

(74) Mandataire: **Lhoste, Catherine**
**L'OREAL-DPI**
**6 rue Bertrand Sincholle**
**92585 Clichy Cédex (FR)**

(54) **Composition aqueuse comprenant un mélange de polyesters sulfonés**

(57)     L'invention a pour objet une composition cosmétique ou dermatologique comprenant dans une phase aqueuse:

i) un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-]  \quad (I)$$

dans laquelle

- A représente un groupement 1,4-phénylène, sulfo-1,3-phénylène,
- n va de 1 à 4,

- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,

la masse moléculaire en poids dudit oligomère copolyester étant inférieure à 20 000,
ii) au moins un copolyester sulfo-isophtalique dispersible dans la phase aqueuse.

Cette composition est destinée au maquillage des matières kératiniques.

## Description

La présente invention concerne une nouvelle composition filmogène sans transfert et résistant à l'eau à application topique, comprenant un mélange de polyesters sulfonés destinée en particulier aux domaines cosmétique et/ou dermatologique. L'invention se rapporte aussi à l'utilisation de cette composition pour le maquillage ou le traitement cosmétique des matières kératiniques d'être humain telles que la peau, les ongles, les cils, les sourcils, les cheveux, ou des muqueuses telles que les lèvres. Cette composition peut se présenter sous forme de gel, de pâte ou de produit coulé notamment de stick.

Les produits de maquillage ou de soin de la peau ou des lèvres comme les fonds de teints ou les rouges à lèvres contiennent généralement des phases grasses telles que des cires et des huiles, des pigments et/ou des charges et, éventuellement des additifs comme des actifs cosmétiques ou dermatologiques. Elles peuvent contenir des gélifiants permettant d'obtenir la consistance souhaitée pour la composition selon son application.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. En outre, l'apparition de ces traces inacceptables notamment sur les cols de chemisier peut écarter certaines femmes de l'utilisation de ce type de maquillage. Il est connu de la demande EP-A-602905 d'associer des huiles volatiles à des cires pour obtenir des produits ayant des propriétés de sans-transfert améliorées. Toutefois, ces produits glissent lors de leur application sur les matières kératiniques, comme par exemple les lèvres, rendant leur application difficile à maîtriser. De plus, ces produits laissent une impression de tiraillement pendant et après leur application sur les lèvres.

Par ailleurs, les compositions après l'application sur la peau ou les lèvres peuvent s'éliminer au contact de l'eau, notamment lors de la baignade ou au contact de la pluie ou des larmes. Il est donc souhaitable de disposer de compositions présentant, en outre, de bonnes propriétés de rémanence à l'eau.

Le but de la présente invention est de proposer une nouvelle composition filmogène aqueuse ne présentant pas les inconvénients évoqués ci-dessus et présentant notamment des propriétés de sans transfert total, de rémanence à l'eau, d'absence de tiraillement pendant et après l'application sur les matières kératiniques, et d'application facile.

Les inventeurs ont découvert qu'une telle composition pouvait être obtenue en utilisant un mélange de polyesters sulfonés. On obtient alors une composition laissant après l'application un film présentant une bonne tenue, ne transférant pas du tout, et résistant à l'eau. Le film est notamment brillant, non collant et confortable (non tiraillement) pendant et après l'application de la composition sur les matières kératiniques. Le film produit également une sensation de fraîcheur et est facile à démaquiller avec les démaquillants habituellement utilisés. De plus, la composition est moins glissante que les produits sans transfert contenant des huiles volatiles, facilitant ainsi son application sur les matières kératiniques.

La présente invention a donc pour objet une nouvelle composition filmogène à application topique comprenant dans une phase aqueuse un agent gélifiant hydrophile de type polyester sulfoné, caractérisée par le fait que l'agent gélifiant hydrophile est un oligomère copolyester téréphtalique hydrosoluble ou hydrodispersible et que la composition comprend également un copolyester sulfoné isophtalique dispersible dans la phase aqueuse.

Les agents gélifiants particuliers utilisés selon l'invention sont des oligomères copolyesters téréphtaliques hydrosolubles ou hydrodispersables comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,

la masse moléculaire en poids desdits oligoméres copolyesters étant inférieure à 20 000, de préférence inférieure à 15 000.

De manière préférentielle, au moins 40 % en mole, et plus préférentiellement entre 40 et 90 % en mole des motifs de formule (I) sont des motifs de formule (I) pour lesquels A représente un groupement 1,4-phénylène et n est égal à 1.

De préférence, au moins 10 % en mole, plus préférentiellement entre 10 % et 25 % en mole des motifs de formule (I) sont des motifs de formule (I) pour lesquels A représente un groupement sulfo-1,3-phénylène.

Les extrémités des chaînes desdits oligomères copolyesters peuvent être semblables ou différentes et représentées essentiellement par les groupements de formule (I'):

$$-CO-A-CO-O-(CH_2-CH_2-O)_n-H \qquad\qquad (I')$$

dans laquelle A et n sont définis ci-dessus.

Lesdits oligomères peuvent également présenter en extrémités de chaîne, et en quantité mineure, des groupements de formules

$$-A-CO-OH$$

$$-A-CO-OR$$

formules dans lesquelles A est défini ci-dessus et R représente un groupement alkyle en $C_1$-$C_4$.

Lorsque A représente un groupement sulfo-1,3-phénylène, il s'agit plus particulièrement d'un sulfonate de métaux alcalins, notamment de sodium ou de potassium, ou d'un sulfonate d'ammonium ou de mono-, di-, tri- ou tétra-alkylammonium inférieur. Par alkylammonium inférieur, on entend de préférence un ammonium dont le ou les radicaux alkyles sont des alkyles inférieurs, de préférence en $C_1$-$C_6$. De manière préférentielle, il s'agit d'un sulfonate de sodium.

L'oligomère copolyester peut comprendre éventuellement jusqu'à 20 % en mole, de préférence de 0,5 à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids allant de 5 000 et 14 000, et plus préférentiellement de 8 000 à 10 000.

Les masses moléculaires en poids sont mesurées par chromatographie par perméation de gel dans le diméthylacé-tamide contenant $10^{-2}$ N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions

.  d'estérification de diacides et de diols et polycondensation

.  de transestérification de diesters et de diols et polycondensation

.  d'autocondensation d'hydroxyacides

.  de Schotten-Baumann par mise en oeuvre de diols et de chlorures d'acide et polycondensation

.  de polymérisation de lactones

en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les oligomères copolyesters téréphtaliques utilisés selon l'invention peuvent être préparés par estérification et/ou transestérification / polycondensation d'une composition monomère à base:

-  d'acide, anhydride ou diester téréphtalique (Tp)

-  d'acide, anhydride ou diester sulfoisophtalique (Slp)

- éventuellement d'acide, anhydride ou diester isophtalique (Ip)
- et d'éthylène glycol (EG)

selon des quantités relatives correspondant à

* un rapport molaire (Slp) / [(Tp)+(Slp)+(lp)] d'au moins 7/100, de préférence d'au moins 10/100, tout particulièrement de 10/100 à 25/100
* un rapport molaire (Ip) / [(Tp)+(Slp)+(lp)] de 20/100 au plus, de préférence de 5/100 au plus
* un rapport molaire (EG) / [(Tp)+(Slp)+(lp)] de 2/1 à 3/1.

en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en $C_1$-$C_4$), de diméthyle de préférence.

Le monomère sulfoisophtalique (Slp) est de préférence mis en oeuvre sous la forme d'un sulfonate de métal alcalin (sodium notamment) de diester inférieur (d'alkyle en $C_1$-$C_4$), de méthyle de préférence. On peut citer tout particulièrement le sodio-oxysulfonyl-5 isophtalate de diméthyle.

Le monomère isophtalique (Ip) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation.

Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2''-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001% à 0,05% par rapport au poids de réactifs présents.

La durée de l'opération d'interéchange est de 1 à 4 heures ; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification.

L'opération est réalisée en présence des mêmes types de catalyseur et de limiteur d'éthérification que ceux mis en oeuvre lors de l'opération de transestérification, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997.

L'oligomère copolyester téréphtalique peut être présent dans la composition selon l'invention en une teneur allant de 0,5 % à 40 % en poids, par rapport au poids total de la composition. Selon la forme recherchée, gélifiée, pâteuse ou solide, on emploiera des quantités différentes de gélifiant particulier. Pour une composition solide, on emploiera de préférence de 10 à 40 % en poids de gélifiant particulier, plus préférentiellement de 15 à 30 % en poids. Pour une composition gélifiée ou pâteuse, on emploiera de préférence de 0,5 à 10 % en poids de gélifiant particulier, plus préférentiellement de 2 à 5 % en poids.

Par copolyester sulfo-isophtalique, on entend dans la présente demande un copolyester consistant essentiellement en des unités répétées d'acide isophtalique, de diol et d'acide sulfo-isophtalique.

Par acide isophtalique, on entend selon la présente demande également les esters et le chlorure d'acide de l'acide isophtalique.

Le diol présent dans le copolyester sulfo-isophtalique peut être un diol cycloaliphatique ayant de préférence de 6 à 20 atomes de carbone ou un diol aliphatique ayant de préférence de 3 à 20 atomes de carbone. Comme diol, on peut utiliser l'éthylène glycol, le diéthylène glycol, le triéthylène glycol, le 1,4-cyclohexanediméthanol, le propane -1,3-diol, le butane-1,4-diol, le pentane-1,5-diol, l'hexane-1,6-diol, le 3-méthylpentanediol-(2,4), le 2-méthylpentanediol-(1,4), le 2,2,4-triméthylpentanediol-(1,3), le 2-éthylhexanediol-(1,3), le 2,2-diéthylpropane-diol-(1,3), l'hexanediol-(1,3), le 1,4-di-(hydroxyéthoxy)-benzène, le 2,2-bis-(4-hydroxycyclohexyl)-propane, le 2,4-dihydroxy-1,1,3,3-tétraméthylcyclobutane, le 2,2-bis-(3-hydroxyéthoxyphényl)-propane, le 2,2-bis-(4-hydroxypropoxyphényl)-propane. Le copolyester isophtalique peut comporter un ou plusieurs diols tels que cités précédemment et de préférence un mélange d'éthylène glycol ou de 1,4-cyclohexanediméthanol et de diéthylène glycol. Avantageusement, le copolyester sulfo-isophtalique peut comporter un mélange de diol consistant en au moins 45 % en mole de diéthylène glycol et en complément à 100 % en mole d'éthylène glycol ou de 1,4-cyclohexane diméthanol.

Le motif acide sulfo-isophtalique comporte un groupement -SO$_3$M avec M représentant un atome d'hydrogè-ne, un ion ammonium NH$_4^+$ ou un ion métallique , comme par exemple Na$^+$, Li$^+$, K$^+$, M étant de préférence Na$^+$. L'acide sulfo-isophtalique particulièrement préféré est le sel de sodium de l'acide 5-sulfo-isophtalique.

Le motif acide sulfo-isophtalique est de préférence présent dans le copolyester sulfo-isophtalique en une teneur pouvant aller de 4 à 25 % en moles pour 100 moles d'acides et 100 moles de diols.

Avantageusement, le copolyester sulfo-isophtalique peut comprendre, pour 100 moles d'acides, de 75 % à 90 % en mole d'acide isophtalique et de 10 % à 25 % en mole d'acide sulpho-isophtalique, notamment de sel de sodium de l'acide 5-sulfoisophtalique, et, pour 100 moles de diols, de 45 % à 85 % en mole de diéthylène glycol et de 15 % à 55 % en mole de 1,4-cyclohexanediméthanol ou d'éthylène glycol ou leur mélange.

Le copolyester sulfo-isophtalique a de préférence une température de transition vitreuse allant de 20 °C à 60 °C, et mieux de 20 °C à 40 °C.

Avantageusement, le copolyster sulfo-isophtalique peut avoir une viscosité inhérente allant de 0,01 à 0,1 litre/ gramme (0,1 à 1 décilitre/gramme), et mieux de 0,02 à 0,05 litre/gramme (0,2 à 0,5 décilitre/gramme), mesurée dans un mélange en poids de phénol et de tétrachloroéthane (60/40) à 25 °C et à une concentration de 0,5 g de polymère pour 100 ml de solvant, selon la norme ASTM D2857-70. La méthode de mesure de la viscosité inhérente est décrite dans le brevet US-A-5260052 dont le contenu est incorporé par référence dans la présente demande.

De tels copolyesters sulfo-isophtaliques sont notamment décrits dans les brevets US-A-3779993 et US-A-5260052 et sont par exemple commercialisés sous les dénominations "EASTMAN AQ 35S", " EASTMAN AQ 38S", " EASTMAN AQ 55S", "EASTMAN AQ 29D", "EASTMAN AQ 38D" par la société EASTMAN CHEMICAL.

La composition selon l'invention peut comprendre un ou plusieurs copolyester sulfo-isophtalique.

Selon l'invention, le copolyester sulfo-isophtalique peut être présent dans la composition en une quantité de matières sèches allant de 1 % à 40 % en poids par rapport au poids total de la composition, et de préférence de 5 % à 20 % en poids.

Avantageusement, l'oligomère copolyester téréphtalique et le copolyester sulfo-isophtalique peuvent être présent dans la composition selon l'invention dans un rapport pondéral oligomère/ copolyester sulfo-isophtalique allant de 0,5 à 20, et de préférence de 1 à 5.

Par ailleurs, la composition selon l'invention peut contenir des adjuvants couramment utilisés dans les compositions cosmétiques, notamment topiques. On peut citer à titre d'exemple d'adjuvants les colorants, les pigments, les nacres, les agents anti-UV, les conservateurs, les agents épaississants, les agents plastifiants, les tensioactifs, les cires, les huiles, les parfums, les agents modificateurs de pH, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être utilisée pour le maquillage, le soin ou le traitement cosmétique non thérapeutique des matières kératiniques et/ou des muqueuses. La composition de maquillage peut être un produit de maquillage des lèvres, notamment sous forme de pâte ou de stick, un fond de teint, un fard à paupières ou à joue, un mascara pour les cils ou les cheveux, un eye-liner, un anti-cernes, ou bien encore une composition de maquillage du corps. La composition de traitement ou de soin cosmétique peut être une composition pour le soin du visage, du cou, des mains, du corps, des ongles ou des lèvres, une composition déodorante ou encore de protection solaire.

L'invention se rapporte également à un procédé non thérapeutique de traitement ou de maquillage des matières kératiniques et/ou des muqueuses consistant à appliquer sur les matières kératiniques et/ou des muqueuses une composition telle que décrite précédemment.

L'invention a également pour objet l'utilisation d'un oligomère copolyester téréphtalique et d'un copolyester sulfo-isophtalique tels que définis précédemment, dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

L'invention a aussi pour objet l'utilisation d'un oligomère copolyester téréphtalique et d'un copolyester sulfo-isophtalique tels que définis précédemment dans une composition cosmétique ou dermatologique pour l'obtention d'un film de cette composition résistant à l'eau et/ou frais et/ou confortable.

L'invention a encore pour objet l'utilisation d'une composition telle que définie précédemment pour l'obtention d'un film de cette composition résistant à l'eau et/ou ayant des propriétés de non transfert et/ou frais et/ou confortable.

On va maintenant donner des exemples illustrant la présente invention sans toutefois la limiter.

### Exemple 1      Préparation d'un oligomère copolyester téréphtalique

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit:

- 11,47 moles de téréphtalate de diméthyle
- 2,53 moles de diméthylisophtalate-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :

- 0,5 mole d'acide isophtalique
- 2,36 moles d'acide téréphtalique
- 8 moles d'éthylène glycol

La masse réactionnelle est alors amenée à la température de 250°C en 60 minutes.
Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.

Le copolyester obtenu présente les caractéristiques structurales décrites au tableau 1 ci-après, dans lequel:

*    "% molaire des motifs diacides" correspond à la teneur, en %, de chaque diacide ou diester mise en oeuvre par rapport à l'ensemble des diacides ou diesters mis en oeuvre.

"Tp" signifie : motif téréphtalique
"Ip" signifie : motif isophtalique
"SIp" signifie : motif sulfoisophtalique

Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.

- "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.

* "%GT/Σ motifs" correspond au % molaire des motifs de formule (I)

$$[-CO-A-CO-O-(CH_2-CH_2-O)_n-] \tag{I}$$

où A est 1,4 phénylène et n=1
par rapport à l'ensemble des motifs de formule (I)
où A est 1,4 phénylène, sulfo 1,3 phénylène et éventuellement 1,3 phénylène et n va de 1 à 4
"%GT/Σ motifs" se calcule par la formule suivante :

$$\%GT/\Sigma \text{ motifs} = (\% \text{ molaire de motifs Tp}) \times (\% \text{ molaire de motifs G}) / 100$$

* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| SIp | 15 |
| %GT/Σ motifs | 46,5 |
| % molaire des motifs diols | |
| G | 56,8 |
| 2G | 30,7 |
| 3G | 10 |
| 4G | 2,5 |
| Mw | 8 000 |

**Exemple 2 :**

On a préparé un rouge à lèvres en forme de stick ayant la composition suivante :

- oligomère copolyester téréphtalique de l'exemple 1     20 %
- copolyester sulfo-isophtalique (EASTMAN AQ 55S de EASTMAN CHEMICAL)     10 % MA
- pigments     5 %
- propylène glycol     2,5 %
- eau     qsp     100 %

On obtient un rouge à lèvres permettant l'obtention d'un film "sans transfert" total et qui résiste parfaitement bien à l'eau.

**Exemple 3:**

On a préparé un rouge à lèvres en forme de stick ayant la composition suivante :

- oligomère copolyester téréphtalique de l'exemple 1     20 %
- copolyester sulfo-isophtalique (EASTMAN AQ 38S de EASTMAN CHEMICAL)     10 % MA
- pigments     5 %

- propylène glycol        2,5 %
- eau        qsp        100 %

On obtient un rouge à lèvres permettant l'obtention d'un film "sans transfert" total et qui résiste parfaitement bien à l'eau.

**Exemple 4:**

On a préparé un mascara en forme de gel ayant la composition suivante :

- oligomère copolyester téréphtalique de l'exemple 1        10 %
- copolyester sulfo-isophtalique (EASTMAN AQ 55S de EASTMAN CHEMICAL)        8 % MA
- pigments        8 %
- propylène glycol        4 %
- eau        qsp        100 %

On obtient un mascara permettant l'obtention d'un maquillage "sans transfert" et qui résiste parfaitement bien à l'eau.

**Exemple 5 :**

On a préparé un produit de maquillage du corps ayant la composition suivante :

- oligomère copolyester téréphtalique de l'exemple 1        5 %
- copolyester sulfo-isophtalique (EASTMAN AQ 38S de EASTMAN CHEMICAL)        5 % MA
- pigments        5 %
- propylène glycol        2,5 %
- eau        qsp        100 %

**Revendications**

1.  Composition filmogène à application topique comprenant dans une phase aqueuse un agent gélifiant hydrophile de type polyester sulfoné, caractérisée en ce que :

    i) le gélifiant hydrophile de type polyester sulfoné est un oligomère copolyester téréphtalique hydrosoluble ou hydrodispersable comprenant essentiellement des motifs répétitifs dicarboxylates de formule (I):

    $$[-CO-A-CO-O-(CH_2-CH_2O)_n-] \qquad (I)$$

    dans laquelle

    - A représente un groupement 1,4-phénylène, sulfo-1,3-phénylène et éventuellement 1,3-phénylène,
    - n va de 1 à 4,
    - au moins 35 % en mole desdits motifs de formule (I) étant des motifs pour lesquels A représente un groupement 1,4-phénylène et n est égal à 1,
    - au moins 7 % en mole desdits motifs de formule (I) étant des motifs de formule (I) pour lesquels A représente un groupement sulfo-1,3-phénylène,
    - et éventuellement jusqu'à 20 % en mole, de préférence jusqu'à 0,5 % à 5 % en mole de motifs de formule (I) pour lesquels A représente un groupement 1,3-phénylène, et

    la masse moléculaire en poids dudit oligomère copolyester étant inférieure à 20 000,
    ii) et que la composition comprend également au moins un copolyester sulfo-isophtalique dispersé dans la phase aqueuse.

2.  Composition selon la revendication 1, caractérisée par le fait que l'oligomère copolyester a une masse moléculaire en poids inférieure à 15 000.

3. Composition selon la revendication 1 ou 2, caractérisée par le fait que l'oligomère copolyester téréphtalique a une masse moléculaire en poids allant de 5 000 à 14 000, et mieux de 8 000 à 10 000.

4. Composition selon l'une quelconque des revendications précédentes, caractérisée par le fait que l'oligomère copolyester téréphtalique comprend au moins 40 % en mole desdits motifs de formule (I) pour lesquels A représente un groupement 1,4-phénylène et n est égal à 1.

5. Composition selon l'une quelconque des revendications précédentes, caractérisée par le fait que l'oligomère copolyester téréphtalique comprend de 40 à 90 % en mole desdits motifs de formule (I) pour lesquels A représente un groupement 1,4-phénylène et n est égal à 1.

6. Composition selon l'une quelconque des revendications précédentes, caractérisée par le fait que l'oligomère copolyester téréphtalique comprend au moins 10 % en mole des motifs de formule (I) pour lesquels A représente un groupement sulfo-1,3-phénylène.

7. Composition selon l'une quelconque des revendications précédentes, caractérisée par le fait que l'oligomère copolyester téréphtalique comprend de 10 % et 25 % en mole desdits motifs de formule (I) pour lesquels A représente un groupement sulfo-1,3-phénylène.

8. Composition selon l'une quelconque des revendications précédentes, caractérisée par le fait que l'oligomère copolyester téréphtalique comprend jusqu'à 20 % en mole, et de préférence de 0,5 % à 5 % en mole, de motifs de formule (I) pour lesquels A représente un groupement 1,3-phénylène.

9. Composition selon l'une quelconque des revendications précédentes, caractérisée par le fait que l'oligomère copolyester téréphtalique est présent en une teneur allant de 0,5 % à 40 % en poids, par rapport au poids total de la composition.

10. Composition selon l'une quelconque des revendications précédentes sous forme solide, caractérisée par le fait que l'oligomère copolyester téréphtalique est présent en une teneur allant de 10 % à 40 % en poids, par rapport au poids total de la composition, et mieux de 15 % à 30 % en poids.

11. Composition selon l'une quelconque des revendications 1 à 9 sous forme de gel, caractérisée par le fait que l'oligomère copolyester téréphtalique est présent en une teneur allant de 0,5 % à 10 % en poids, par rapport au poids total de la composition, et mieux de 2 % à 5 % en poids.

12. Composition selon l'une quelconque des revendications précédentes, caractérisée par le fait que le copolyester sulfo-isophtalique consiste essentiellement en des unités répétées d'acide isophtalique, de diol et d'acide sulfo-isophtalique.

13. Composition selon la revendication 12, caractérisée par le fait que le diol est choisi dans le groupe formé par l'éthylène glycol, le diéthylène glycol, le triéthylène glycol, le 1,4-cyclohexanediméthanol.

14. Composition selon l'une quelconque des revendications précédentes, caractérisée par le fait que le copolyester sulfo-isophtalique comprend un mélange de diol consistant en au moins 45 % en mole de diéthylène glycol et en complément à 100 % en mole d'éthylène glycol ou de 1,4-cyclohexane diméthanol.

15. Composition selon l'une quelconque des revendications 12 à 14, caractérisée par le fait que le motif acide sulfo-isophtalique est le sel de sodium de l'acide 5-sulfo-isophtalique.

16. Composition selon l'une quelconque des revendications 12 à 15, caractérisée par le fait que le motif acide sulfo-isophtalique est présent dans le copolyester sulfo-isophtalique en une teneur allant de 4 % à 25 % en moles pour 100 moles d'acides et 100 moles de diols.

17. Composition selon l'une quelconque des revendications précédentes, caractérisée par le fait que le copolyester sulfo-isophtalique comprend, pour 100 moles d'acides, de 75 % à 90 % en mole d'acide isophtalique et de 10 % à 25 % en mole d'acide sulpho-isophtalique et, pour 100 moles de diols, de 45 % à 85 % en mole de diéthylène glycol et de 15 % à 55 % en mole de 1,4-cyclohexanediméthanol ou d'éthylène glycol ou leur mélange.

18. Composition selon l'une quelconque des revendications précédentes, caractérisée par le fait que le copolyester sulfo-isophtalique a une température de transition vitreuse allant de 28 °C à 60 °C.

19. Composition selon l'une quelconque des revendications précédentes, caractérisée par le fait que le copolyster sulfo-isophtalique a une viscosité inhérente allant de 0,01 à 0,1 litre/gramme, et mieux de 0,2 à 0,5 litre/gramme, mesurée dans un mélange en poids de phénol et de tétrachloroéthane (60/40) à 25 °C et à une concentration de 0,5 g de polymère pour 100 ml de solvant.

20. Composition selon l'une quelconque des revendications précédentes, caractérisée par le fait que le copolyester sulfo-isophtalique est présent en une quantité de matières sèches allant de 3 % à 50 % en poids par rapport au poids total de la composition.

21. Composition selon l'une quelconque des revendications précédentes, caractérisée par le fait que l'oligomère copolyester téréphtalique et le copolyester sulfo-isophtalique sont présents selon un rapport pondéral oligomère/copolyester sulfo-isophtalique allant de 0,5 à 20, et mieux de 1 à 5.

22. Composition selon l'une quelconque des revendications précédentes, caractérisée par le fait que la composition est une composition cosmétique ou dermatologique.

23. Composition cosmétique selon l'une quelconque des revendications précédentes, caractérisée par le fait qu'elle comprend au moins un adjuvant choisi dans le groupe formé par les colorants, les pigments, les nacres, les agents anti-UV, les conservateurs, les agents épaississants, les agents plastifiants, les tensioactifs, les cires, les huiles, les parfums, les agents modificateurs de pH, les agents hydratants.

24. Composition selon l'une quelconque des revendications précédentes, caractérisée par le fait qu'elle se présente sous forme de composition de maquillage ou de traitement cosmétique des matières kératiniques et/ou des muqueuses.

25. Composition selon l'une quelconque des revendications précédentes, caractérisée par le fait que la composition se présente sous la forme d'un produit de maquillage des lèvres, d'un fond de teint, d'un fard à paupières ou à joue, d'un mascara, d'un eye-liner, d'un anti-cernes, d'une composition de maquillage du corps, d'une composition pour le soin du visage, du cou, des mains, du corps, des ongles ou des lèvres, d'une composition déodorante, d'une composition de protection solaire.

26. Procédé non thérapeutique de maquillage ou de traitement des matières kératiniques et/ou des muqueuses, caractérisé par le fait que l'on applique sur les matières kératiniques et/ou des muqueuses une composition selon l'une des revendications 1 à 24.

27. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un copolyester sulfo-isophtalique dispersible dans l'eau,
dans une composition cosmétique ou dermatologique pour diminuer, voire supprimer, le transfert du film de cette composition déposé sur les matières kératiniques et/ou les muqueuses, et/ou pour supprimer le dépôt de traces sur un autre support autre que lesdites matières kératiniques et/ou lesdites muqueuses .

28. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un copolyester sulfo-isophtalique dispersible dans l'eau,
dans une composition cosmétique ou dermatologique pour l'obtention d'un film de cette composition résistant à l'eau et/ou frais et/ou confortable.

29. Utilisation d'une composition selon l'une quelconque des revendications 1 à 25 pour l'obtention d'un film résistant à l'eau et/ou ayant des propriétés de non transfert et/ou frais et/ou confortable.

**EP 0 997 138 A1**

**RAPPORT DE RECHERCHE EUROPEENNE**

Numéro de la demande

EP 99 40 1777

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | WO 97 45094 A (EASTMAN CHEMICAL COMPANY) 4 décembre 1997 (1997-12-04)<br><br>* revendications 1,2,5,6,8 *<br>* page 6, ligne 19 - page 8, ligne 16 *<br>* page 11, ligne 27 - page 12, ligne 24 *<br>* exemples 1,2 *<br>--- | 1,12-17, 19,20, 22-25 | A61K7/48 A61K7/02 |
| A | WO 97 24102 A (EASTMAN CHEMICAL COMPANY) 10 juillet 1997 (1997-07-10)<br>* revendications 1,2,11,14-19 *<br>* page 9, ligne 24-31 *<br>* page 13, ligne 12-26 *<br>* page 14, ligne 10-13 *<br>* exemple 1 *<br>----- | 1,12-20, 22-25 | |

| | | | DOMAINES TECHNIQUES RECHERCHES (Int.Cl.7) |
|---|---|---|---|
| | | | A61K |

Le présent rapport a été établi pour toutes les revendications

| Lieu de la recherche | Date d'achèvement de la recherche | Examinateur |
|---|---|---|
| LA HAYE | 14 décembre 1999 | Peeters, J |

EPO FORM 1503 03.82 (P04C02)

**ANNEXE AU RAPPORT DE RECHERCHE EUROPEENNE**
**RELATIF A LA DEMANDE DE BREVET EUROPEEN NO.**          EP 99 40 1777

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-12-1999

| Document brevet cité au rapport de recherche | | Date de publication | Membre(s) de la famille de brevet(s) | | Date de publication |
|---|---|---|---|---|---|
| WO 9745094 | A | 04-12-1997 | US | 5744129 A | 28-04-1998 |
| | | | EP | 0906079 A | 07-04-1998 |
| WO 9724102 | A | 10-07-1997 | US | 5925336 A | 20-07-1999 |
| | | | BR | 9612280 A | 13-07-1999 |
| | | | EP | 0869765 A | 14-10-1999 |

EPO FORM P0460

Pour tout renseignement concernant cette annexe : voir Journal Officiel de l'Office européen des brevets, No.12/82